# EUROPEAN PATENT SPECIFICATION

(11) **EP 3 785 733 B1**
(45) Date of publication and mention of the grant of the patent: **05.01.2022**
(21) Application number: 20195373.4
(22) Date of filing: 06.11.2018
(51) Int. Cl.: A61K 45/00, A61P 25/00, A61K 31/196, A61K 31/437

(54) **PHARMACEUTICAL COMPOSITION FOR THE TREATMENT OF AUTISM**
PHARMAZEUTISCHE ZUSAMMENSETZUNG ZUR BEHANDLUNG VON AUTISMUS
COMPOSITIONS PHARMACEUTIQUES POUR LE TRAITEMENT DE L'AUTISME

(30) Priority: 06.11.2017 US 201762582141 P; 06.11.2017 EP 17200219; 27.04.2018 US 201862663647 P; 27.04.2018 EP 18169952
(43) Date of publication of application: 03.03.2021
(62) Divisional of application: 18204763.9
(73) Proprietor: Stalicla S.A., 1202 Geneva (CH)
(72) Inventor: Durham, Lynn, 1202 Genève (CH)
(74) Representative: Stolmár & Partner Intellectual Property GmbH

(56) References cited:
- LEMONNIER ET AL: "Effects of bumetanide on neurobehavioral function in children and adolescents with autism spectrum disorders", TRANSL PSYCHIATRY,, vol. 7, 14 March 2017 (2017-03-14), pages 1-9, XP002789821, DOI: 10.1038/TP.2017.10

## Description

### Field of the invention

The invention relates to a pharmaceutical composition for treating the core symptoms of autism spectrum disorder (ASD) and/or associated symptoms including learning disabilities, language impairment and impairments in executive functioning that can be associated with the disease in patients with ASD - and preferably in phenotype 1 patients - a subcategory of patients presenting with specific differentiating clinical sets of signs and symptoms.

In particular, the composition comprises a substance capable of raising intracellular cAMP levels and a substance capable of modulating intracellular calcium concentrations, wherein the substance capable of raising intracellular cAMP levels is ibudilast and the substance capable of modulating intracellular calcium concentration is bumetanide.

### Background of the invention

Autism spectrum disorder (ASD) are a group of neurodevelopmental disorders frequently characterized by impairments in social interactions, difficulties with language and communication, and the presence of repetitive, perseverative behaviors (Abrahams BS, Geschwind DH; Advances in autism genetics: on the threshold of a new neurobiology; Nat Rev Genet. 2008 Jun;9(6):493), (Zoghbi HY, Bear MF; Synaptic dysfunction in neurodevelopmental disorders associated with autism and intellectual disabilities; Cold Spring Harb Perspect Biol. 2012 Mar 1;4(3). ASD typically appears during the first three years of life and manifests in characteristic symptoms or behavioral traits. A diagnosis of ASD currently includes several conditions that used to be diagnosed separately: autistic disorder, pervasive developmental disorder not otherwise specified (PDD-NOS), and Asperger syndrome. All of these conditions are now encompassed by the diagnostic criteria for autism spectrum disorder as set forth in the American Psychiatric Association's Diagnostic & Statistical Manual of Mental Disorders, Fifth Edition (DSM-5).

While ASD is currently defined by symptoms in core areas, there exists significant heterogeneity in genetics, phenotypes, clinical presentation, and associated comorbidities (Persico AM, Bourgeron T; Searching for ways out of the autism maze: genetic, epigenetic and environmental clues; Trends Neurosci. 2006 Jul;29(7):349-358).

The genetic contribution to the causation/predisposition to autism is considered to be substantial on the basis of high concordance in monozygous twins (Folstein S. Rutter M; Infantile autism: a genetic study of 21 twin pairs; J Child Psychol Psychiatry; 1977 Sep;18(4): 297-321.). A recently published reanalysis of data from a previous study on the familial risk for autism spectrum disorder (ASD) further supports these initial findings suggesting that genetics contribute 83% of the risk for ASD. Environmental factors thus seem to play a minor 17% though significant role in the developmental etiology of ASD (Sandin S, Lichtenstein P, Kuja-Halkola R, Hultman C, Larsson H, Reichenberg A. The Heritability of Autism Spectrum Disorder. JAMA. 2017;318(12):1182-1184. doi:10.1001/jama.2017.12141.). However, to further complexify matters genetic and epigenetic factors intertwine with prenatal and lifelong dynamic environmental factors to draw individual patient pathogenesis.

There is growing perception among the scientific community that the current behavioral based approaches to diagnostic do not allow for efficient classification of patients in terms of molecular and genetic alterations, but rather serve as a behavioral umbrella term for a large group of neurodevelopmental disorders with different etiologies. Recent developments of new genetic screening methods (e.g., microarray-based, comparative genomic hybridization assay (a-CGH), whole genome or exome sequencing technics...) have permitted to detect hundreds of genetic risk factors, including common and rare genetic variants, which can increase the likelihood of ASD (Ronemus M. et al; The role of the novo mutations in the genetics of autism spectrum disorders; Nat Rev Genet. 2014 Feb; 15(2): 133-41). Nevertheless, causal genetic factors can only be identified in 15 to 20% of patients. Thus the vast majority ASD patients are still considered idiopathic.

Evidence has recently accumulated to support the theory that the ever-expanding number of ASD susceptibility genes do in fact seem to converge towards a limited number of molecular pathways. This growing assumption offers important translational opportunities as molecular pathways mediating synaptic and circuit formation are also involved in other physiological processes including modulation of the adaptive and innate immune response (Myka L. Estes ML, McAllister AK (2015), Nature Reviews Neuroscience 16, 469-486), cell proliferation, survival and protein synthesis (Subramanian M, Timmerman CK, Schwartz JL, Pham DL and Meffert MK (2015), Front. Neurosci. 9:313. Tang G. et al. (2014), Neuron. 83, 1131-1143.).

A subgroup of ASD patients, so called ASD phenotype 1 (see EP 17200185.1) show an upregulation of pathways involved in adaptation to stress, apoptosis or cell differentiation, cell proliferation, cell cycle progression, cell division and differentiation (in particular but not limited to PI3K, AKT, mTOR, MAPK, ERK/JNK-P38). Proinflammatory cytokines (TNF-α, IL-6, IL-1β, IL-17A, IL-22 and GM-CSF), Th1 cytokine (INF-y) and chemokine (IL-8) may also be significantly increased in the brain of these patients compared to healthy control patients. Th2 cytokines (IL-4, IL-5) and IL-10 may not show significant differences. The Th1/Th2 ratio may be thus be significantly increased in ASD Phenotype 1 patients.

Currently, there are no effective treatment for this subgroup of patients. In fact, these patients react negatively to administration of antioxidant substances, despite the fact that these have been reported to improve some patients with autism.

Fragile X is the most common monogenetic cause of both intellectual disability and autism spectrum disorder. Patients with Fragile X syndrome (FXS), caused by loss of function of the fragile X mental retardation 1 (*Fmr1*) gene, often exhibit many of the symptoms commonly associated with ASD, such as developmental delays, communication impairments and anxiety. These overlaps have led many investigators to conclude that *Fmr1* knockout mice provide a unique opportunity to identify interventions that affect autistic-like behaviors (Mines MA et al; GSK3 influences social preference and anxiety related behaviors during social interaction in a mouse model of fragile X syndrome and autism. PLoS One. 2010 Mar 16;5(3): e9706). Studies of Fragile X models have found low levels of cyclic Adenosine Monophosphate (cAMP) in the brains of both *dfmr1* mutant flies and *fmr1* knockout (KO) mice (Kelly DJ. et al.; The cyclic AMP cascade is altered in the fragile X nervous system; PLoS One. 2007 Sep 26;2(9): e931. Choi et al.; PDE-4 inhibition rescues aberrant synaptic plasticity in Drosophila and mouse model of fragile X syndrome; J. Neurosci 2015. 35, 396-408).

Decreased cAMP levels have also been demonstrated in Fragile X patients (Berry-Kravis E. et al.; Cyclic AMP metabolism in fragile X syndrome; Ann. Neurol. 1992. 31, 22-26. Berry-Kravis E. et al.; Reduced cyclic AMP production in fragile X syndrome: cytogenetic and molecular correlation. Pediatr. Res. 1995. 38, 638-643. Berry-Kravis E. et al.; Overexpression of fragile X gene (FMR-1) transcripts increase cAMP production in neuronal cells. J. Neurosci. Res. 1998. 51, 41-48).

Cyclic Adenosine Monophosphate (cAMP) is synthetized from adenosine triphosphate (ATP) via the action of adenylyl cyclase (AC) and is converted to its inactive form 5'- adenosin monophosphate (5'-AMP) via hydrolysis by phosphodiesterase (PDE) (Maurice DH et al. Cyclic nucleotide phosphodiesterase activity, expression and targeting in cells of the cardiovascular system. Mol Pharmacol. 2003. 64: 533-546). As a result of the degradation of cAMP by PDE, the catalytic portion of protein kinase A (PKA) is effectively prevented from translocating to the nucleus and generating phosphorylated-cAMP response element-binding protein (p-CREB) (McLean JH et al. A phosphodiesterase inhibitor, cilomilast, enhances cAMP activity to restore conditioned odor preference memory after serotonergic depletion in the neonate rat. Neurobiol Learn Mem. 2009. 92: 63-69). cAMP regulates numerous cellular functions, including metabolism, transcription and growth, in the majority of cell types. These cAMP effects, mediated primarily by cAMP-dependent PKA, are at the root of cAMP-mediated regulation of various physiological processes, including endocrine, cardiovascular, neuronal and immune functions (Jackson et al. Role of the extracellular cAMP-adenosine pathway in renal physiology. AM J Physiol Renal Physiol 281: F597-F612,2001. Seino S et al. PKA-dependent and PKA-independent pathways for cAMP-regulated exocytosis. AM J Physiol Rev 85: 1303-1342, 2005. Richards JS. New signalling pathways for hormones and cyclic adenosine 3',5'-monophosphate action in endocrine cells. Mol Endocrinol 15: 209-218, 2001).

Interestingly, *FMR1* transcription is dependent on CREB mediated transcription in flies and mammals, which is activated by the cAMP pathway (Hwu et al.; FMR1 enhancer is regulated by cAMP through a cAMP-responsive element. DNA Cell Biol. 1997, 16, 449- 453); (Cha-Molstad et al.; Cell-type specific binding of the transcription factor CREB to the cAMP-response element. Pro. Natl. Acad. Sci. U.S.A. 2004; 101, 13572-13577. Kanellopoulos et al.; Learning and memory deficits consequent to reduction of the fragile X mental retardation protein result from metabotropic glutamate receptor-mediated inhibition of cAMP signalling in Drosophila; J. Neurosci. 2012. 32,13111-13124) and consistent with these findings there is a positive correlation between FMRP levels and cAMP levels in cell lines and platelets derived from afflicted patients (Berry-Kravis E. et al.; Cyclic AMP metabolism in fragile X syndrome; Ann. Neurol. 1992. 31, 22-26. Berry- Kravis E. et al.; Reduced cyclic AMP production in fragile X syndrome: cytogenetic and molecular correlation. Pediatr. Res. 1995. 38, 638-643. Berry-Kravis E. et al.; Overexpression of fragile X gene (FMR-1) transcripts increase cAMP production in neuronal cells. J. Neurosci. Res. 1998. 51, 41-48).

This led to the hypothesis that one way to correct some phenotypic cognitive deficits in Fragile X syndrome is to increase cAMP to wild type physiological levels.

It has previously been demonstrated that treatment of the Fragile X fly model with PDE-4 inhibitors could rescue social interaction, immediate recall memory and short-term memory (McBride et al., Pharmacological rescue of synaptic plasticity, courtship behaviour, and mushroom body defects in a Drosophila model of fragile X syndrome. Neuron. 2005. 45, 753-764; Bolduc et al.; Excess protein synthesis in drosophila fragile X mutants impairs long-term memory. Nat. Neurosci. 2008. 11, 1143-1145. Choi et al.; PDE-4 inhibition rescues aberrant synaptic plasticity in Drosophila and mouse models of fragile X syndrome. J. Neurosci. 2015. 35, 396-408).

One type of cAMP-PDEs is the PDE4 family which comprises four genes, PDE4A to D. PDE4s differ from other cAMP-PDEs by their kinetic properties and, particularly, their sensitivity to inhibition by the prototypical PDE4 inhibitor rolipram. Soon after their discovery more than 30 years ago, several lines of evidence emerged indicating a role for PDE4s in regulating brain function.

Ibudilast has been previously tested in an experimental mouse model of ASD (Coiro P. et al., "Impaired synaptic development in a maternal immune activation mouse model of neurodevelopmental disorders", Brain, behavior, and immunity. 2015. 50: 249 - 258).

In addition to affecting active cognitive mechanisms, PDE4 inhibition may prevent disruption of brain neurobiological homeostasis that results in cognitive dysfunction. This includes anti-inflammatory effects (Wito Richter et al.; PDE4 as a target for cognition enhancement; Expert Opin Ther Targets 2013 Sep; 17(9): 1011-1027). Three PDE-4 inhibitors are currently available clinically: 1) Apremilast approved for psoriatic arthritis and plaque psoriasis; 2) Roflumilast approved for the treatment of chronic obstructive pulmonary disease (COPD); and 3) Ibudilast approved for the treatment of asthma and cerebrovascular disorders (including post-stroke complications). Whereas Apremilast and Roflumilast have poor blood brain barrier penetration (Kavanaugh et al.; Longterm (52-week) results of a phase III randomized, controlled trial of apremilast in patients with psoriatic arthritis. J. Rheumatol. 2015. 42, 479-488); (Martinez et al.; Effect of roflumilast on exacerbations in patients with severe chronic obstructive pulmonary disease uncontrolled by combination therapy (REACT): a multicentre randomised controlled trial. Lancet 385, 857-866. 2015), Ibudilast has been demonstrated to cross the blood brain barrier (Ledeboer A et al. Ibudilast (AV-411). A new class therapeutic candidate for neuropathic pain and opioid withdrawal syndromes. Expert Opin Investig Drugs, 2007 Jul;16(7):935-50. Poland et al. Ibudilast attenuates expression of behavioral sensitization to cocaine in male and female rats. Neuropharmacology, 2016 October; 109: 281-292).

However, there are currently no treatments for ASD exploiting the connection between intracellular cAMP levels and ASD.

### Objective problem to be solved

The objective problem to be solved is thus the provision of novel treatments for ASD and, in particular, to subgroups of ASD patients characterized by low levels of intracellular cAMP. Because of common genetic and molecular alterations, these treatments may also be useful for the treatment of Fragile X syndrome.

### Summary of the invention

The present invention solves this problem by providing a pharmaceutical composition comprising
∘ a substance capable of raising intracellular cAMP levels and
∘ a substance capable of modulating intracellular calcium concentration,
wherein the substance capable of raising intracellular cAMP levels is ibudilast and the substance capable of modulating intracellular calcium concentration is bumetanide.

Likewise, the problem is solved by a pharmaceutical composition or kit for use in the treatment of ASD phenotype 1, the pharmaceutical composition comprising a substance capable of raising intracellular cAMP levels, wherein the substance capable of raising intracellular cAMP levels is ibudilast and the substance capable of modulating intracellular calcium concentration is bumetanide.

### Detailed description of the invention

In one aspect, the present invention relates to a pharmaceutical composition comprising
∘ a substance capable of raising intracellular cAMP levels and
∘ a substance capable of modulating intracellular calcium concentration,
wherein the substance capable of raising intracellular cAMP levels is ibudilast and the substance capable of modulating intracellular calcium concentration is bumetanide.

A kit is herein defined as combination product provided as a package and containing several individual parts that show a complementary effect when applied together. In this aspect, the effect achieved by a kit and a pharmaceutical composition are similar. A kit offers the advantage that dosage regimens of the individual parts may be adjusted to specific requirements and over time.

As used herein, the term autism spectrum disorder (ASD) is understood to cover a family of neurodevelopmental disorders characterized by deficits in social communication and interaction and restricted, repetitive patterns of behavior, interests or activities. In the following, the terms "autism spectrum disorder", "autism" and "ASD" are used interchangeably.

Herein, the terms "ASD phenotype 1" and "phenotype 1" are used interchangeably. The term "patient" refers to "ASD patient" and is intended to cover not only humans diagnosed as having ASD, but also humans suspected of having ASD.

The person skilled in the art is well aware of how a patient may be diagnosed with ASD.

For example, the skilled person may follow the criteria set up in "American Psychiatric Association; Diagnostic and Statistical Manual of Mental Disorders (DSM-5) Fifth edition" to give a subject a diagnosis of ASD. Likewise, ASD patients may have been diagnosed according to standardized assessments tools including but not limited to CIM-10, ICD-10, DISCO, ADI-R, ADOS or CHAT.

In other cases, patients may have a well-established DSM-IV diagnosis of autistic disorder, Asperger's disorder, or pervasive developmental disorder not otherwise specified (PDD-NOS).

Additionally, the present invention may be useful for subjects fulfilling one or more of the following criteria: persistent deficits in social communication and social interaction across multiple contexts as manifested by the following, currently or by history; restricted, repetitive patterns of behavior, interests, or activities, as manifested by at least two of the following, currently or by history; symptoms present in the early development period (but may not become fully manifest until social demands exceed limited capacities, or maybe masked by learned strategies in later life); symptoms cause clinically significant impairment in social, occupational, or other important areas of current functioning; these disturbances are not better explained by intellectual disability (intellectual development disorder) or global development delay.

ASD may occur with or without accompanying intellectual and/or language impairment. It may be associated with a known medical or genetic condition or an environmental factor or other neurodevelopmental, mental or behavioral disorders.

ASD may occur in different severity levels which may be classified according to impairment in social communication and in terms of restricted, repetitive behavior. Importantly, the term ASD phenotype 1 is not associated with a particular severity level of ASD. The present invention may be applied to patients suffering from any severity level of ASD.

Without being bound by a mechanism, it is believed that ASD patients can be characterized depending on whether or not they show an upregulation, a downregulation or normal levels of expression of biomolecular pathways involved in stress response.

Depending on whether and how the level of expression of these pathways in the respective individuals are modified, challenging ASD patients with Nrf2-activators which are known to upregulate the respective pathways will improve or worsen ASD symptoms.

In one aspect, the pharmaceutical composition or kit according to the present invention is for use in the treatment of an ASD or of a subgroup of ASD patients called ASD phenotype 1 patients.

ASD phenotype 1 patients may be identified with the help of a challenge test as described in non-published EP17200185.1. Briefly, the concept of a challenge test is based on administration of an Nrf2-activator to an ASD patient. In ASD phenotype 1 patients, who already show an upregulation of the respective pathways, further activation of Nrf2 will lead to a worsening of core symptoms. Consequently, ASD phenotype 1 patients may be identified by a negative behavioral response to a challenge test.

Likewise, the skilled person can identify ASD phenotype 1 patient according to the clinical signs as defined in EP 17200185.1.

Another way of identifying ASD phenotype 1 patients is to check for upregulation of Nrf2. The person skilled in the art is well aware of how upregulation of the expression of a specific gene such as Nrf2 may be investigated. For example, it may be investigated at the mRNA level using quantitative PRC techniques such as qPCR or RT-qPCR. Likewise, upregulation of the expression of a gene may be determined on the protein level using protein quantification techniques such as Western Blot and quantitative dot blots. Upregulation is understood to mean an increase of mRNA levels or protein levels of at least 10% when compared to samples from healthy subjects.

Additionally, an ASD phenotype 1 patient may exhibit one or more of the following symptoms: increased levels of TH1 and pro-inflammatory sera cytokines (including but not limited to TNF-α, IL-1β, IL-6, II-17A and IL-22) and/or of tissue expressions of mRNAs that encode inflammatory cytokines and/or excessive challenged T cell secretion of proinflammatory cytokines; upregulation of pathways involved in adaptation to stress, apoptosis, cell differentiation, cell proliferation, cell cycle progression, cell division and differentiation (in particular but not limited to PI3K, AKT, mTOR/MAPK, ERK/JNK-P38).

The pharmaceutical composition or kit according to the present invention comprises a substance that is capable of raising intracellular cAMP levels. A substance may be capable of raising intracellular cAMP levels directly or indirectly or both. A substance capable of directly raising intracellular cAMP levels may directly stabilize cAMP or inhibit degradation of cAMP. A substance capable of indirectly raising intracellular cAMP levels may activate molecules which lead to the generation of cAMP or may inhibit molecules degradating cAMP.

Inhibition is herein understood to mean blocking or reducing the activity of the respective molecule. In some embodiments, activity may be reduced by more than 20%, 30%, 40%, 50%, 60%, 70%, 80% or 90%. In a preferred embodiment, activity is reduced by at least 50%.

Activation is herein understood to mean upregulating or enhancing the activity of the respective molecule. In some embodiments, activity may be enhanced by more than 20%, 30%, 40%, 50%, 60%, 70%, 80% or 90%. In a preferred embodiment, activity may be enhanced by at least 50%.

Substances that are capable of indirectly raising intracellular cAMP levels include substances capable of stimulating a G protein-coupled adenosine Aₐ₂ receptor which activates adenylate cyclase, substances capable of activation of adenylate cyclase, substances that are capable of inhibiting glycogen synthetase kinase-3-beta (GSK-3-beta), as well as substances capable of inhibition of phosphodiesterase.

More specifically, a substance capable of activation of G protein-couple adenosine Aₐ₂ receptor may be a purine nucleoside, in particular regadenoson, bidenoson, adenosine, 2-phenilaminoadenosine, 2-amino-4-(4-hydroxyphenyl)-6-[(1H-imidazol-2-ylmethyl)thio]-3,5-pyridinecarbonitrile, 4-[2-[(6-amino-9-b-D-ribofuranosyl-9H-purin-2-yl)thio]ethyl]benzenesulfonic acid ammonium salt, 2-hexynyl-5'-N-ethylcarboxamidoadenosine, CGS-21680 or UK-432,097.

Substances capable of inhibiting glycogen synthetase kinase-3-beta (GSK-3-beta) may be lithium salts.

Substances capable of inhibition of phosphodiesterase may be any molecule having a c-AMP-specific 3'-5'-cyclic phosphodiesterase 4A/B/C inhibitory effect, any molecule having a c-AMP 3'-5'-cyclic phosphodiesterase 4A/B inhibitory effect, any molecule having a cAMP 3'-5'-cyclic phosphodiesterase 4A inhibitory effect, any molecule having a cAMP 3'-5'-cyclic phosphodiesterase 4A/B/C/D inhibitory effect, any molecule having a cAMP 3'-5'-cyclic phosphodiesterase 4A/B/D inhibitory effect, any molecule having a cAMP 3'-5'-cyclic phosphodiesterase 4/B/D inhibitory effect, any molecule having a cAMP 3'-5'-cyclic phosphodiesterase 4D inhibitory effect, any molecule having a cAMP 3'-5'-cyclic phosphodiesterase 4B/D inhibitory effect, any molecule having a cAMP 3'-5'-cyclic phosphodiesterase 4B inhibitory effect.

Substances capable of inhibition of PDE include pyrazopyridines, preferably ibudilast, xanthines and derivatives, in particular enprofylline, pentoxifylline, dyphylline, aminophylline, propentofylline, preferably caffeine, theobromine, theophilline; flavonoids, in particular xanthohumol, leucocyanidol, delphinidin; flavones, in particular apigenin, luteolin; biflavones, in particular podocarpusflavone A; sequoiaflavones, in particular podocarpusflacvone B, 7,7"-dio-methylaminoflavone, bilobetin; flavanones, in particular dioclein, naringenin, hesperetin; stilbens, in particular E-(epsilon)-viniferin, curcumin; alkaloids, in particular chelerythrine, glaucine, apomorphine; aminopyrimidines and derivatives, in particular diakylarylamines, preferably dipyridamole; arenecarboxamides in partiucular benzamides, preferably roflumilast or piclamilast; benzoxaboroles, in particular crisaborole; isoindolones, in particular phthalimides, preferably apremilast; methoxybenzenes, in particular cilomilast; pyridinecarboxylic acids, in particular tetomilast; phenylpyrrolidines, in particular pyrrolidin-2-ones, preferably rolipram, (S)-rolipram, (R)-rolipram; bipyridines; oligopyridines, in particular amrinone, milrinone; arylphenylketones, in particular enoximone; oxazolidin-2-ones, in particular daxalipram (R-mesopram); probiotics, in particular L-reuteri.

Substance capable of inhibiting PDE include ibudilast, caffeine, theobromine, theophylline, enprofylline, pentoxifylline, dyphylline, L-reuteri, dipyridamole, cilostazol, etazolate, roflumilast, crisaborole resembrenone, drotaverin, apremilast, cilomilast, tetomilast, rolipram, (S)-rolipram, (R)-rolipram, amrinone, milrinone, enoximone, daxalipram (R-mesopram), lirimilast, AWD-12-281, cipamfylline, oglemilast, tofimilast, CI-1044 ((R)-N-(9-amino-4-oxo-1-phenyl-3,4,6,7-tetrahydro-[1,4]diazepino[6,7,1-hi]indol-3-yl)nicotinamide), HT-0712 ((3S,5S)- 2-Piperidinone, 5-(3-(cyclopentyloxy)-4-methoxyphenyl)-3-((3-methylphenyl)methyl), MK-0873 (3-(2-{3-[3-(cyclopropylcarbamoyl)-4-oxo-1,4-dihydro-1,8-naphthyridin-1-yl]phenyl}ethynyl)pyridin-1-ium-1-olate), arofylline, Cl-1018 (N-(3,4,6,7-tetrahydro-9-methyl-4-oxo-1-phenylpyrrolo(3,2,1-jk)(1,4)benzodiazepin-3-yl)-4-Pyridinecarboxamide), T-2585 (2-{4-2,3-bis(hydroxymethyl)-6,7-diethoxy-1-naphthalenyl}-2-pyridinyl]-4-(3-pyridinyl)-1(2H)-phthalazinone), YM-976 (4-{3-chlorophenyl)-1,7-diethyl-1H,2H-pyrido[2,3-d]pyrimidin-2-one), V-11294A (3-(3-Cyclopentyloxy-4-methoxy-benzyl)-8-isopropyl-adenine), piclamilast, atizoram, filaminast, SCH 351591 (N-(3,5-Dichloro-1-oxido-4-pyridinyl)-8-methoxy-2-(trifluoromethyl)-5-quinolinecarboxamide), IC-485, D-4418 (N-(2,5-dichloro-3-pyridinyl)-8-methoxy-5-quinolinecarboxamide), CDP-840 (4-[(2R)-2-[3-(Cyclopentyloxy)-4-methoxyphenyl]-2-phenylethyl]-pyridine hydrochloride), L-826,141 (4-(2-(3,4-bis(difluoromethoxy)phenyl)-2-(4-(1,1,1,3,3,3-hexafluoro-2-hydroxypropan-2-yl)phenyl)ethyl)-3-methylpyridine 1-oxide), BPN14770 (2-(4-((2-(3-chlorophenyl)-6-(trifluoromethyl)pyrimidin-4-yl)amino)phenyl)acetamide), TDP101.

According to the invention, the substance capable of inhibiting PDE is ibudilast.

Ibudilast is an anti-inflammatory and neuroprotective oral agent, metabolized mainly by the liver. Following administration of a single dose of 10 mg ibudilast to healthy adults, about 60% of the dose was excreted as metabolites in urine in 72 hours. The clinical efficacy of this product has been proven for bronchial asthma indication and cerebrovascular disorders. Ibudilast is currently under clinical trial in the U.S. for progressive multiple sclerosis and other conditions such as amyotrophic lateral sclerosis and substances dependence (codes: AV-411 or MN-166).

The pharmaceutical composition or kit according to the present invention also comprises a substance capable of modulating intracellular calcium levels.

Herein, the term "modulating" may refer to a global decrease of cytosolic intracellular calcium, a localized increase of intracellular calcium in specific organelles, such as the mitochondria or endoplasmic reticulum, which in turns lead to a decrease of the cytosolic calcium concentration, or a modulation of the dynamics of intracellular calcium, such as a modulation of intracellular calcium vague, intracellular calcium oscillations, and/or intracellular calcium sparks (Giorgi et al. Trends Cell Biol. 2018, Feb 3;S0962-8924, Calcium Dynamics as a Machine for Decoding Signals).

A substance capable of modulating intracellular calcium levels may modulate intracellular calcium levels by mechanisms including either inhibition of intracellular calcium channels such as ryanodine receptor (RyR) or inositol-1,4,5-triphosphate-receptor (IP3R), or direct or indirect modulation of calcium-ATPase pumps, including Sarcoendoplasmic reticulum Ca2+ transport-ATPase (SERCA) and Plasma Membrane Ca2+ ATPase (PMCA), ionic exchangers, such as Na+/Ca2+ exchangers, Na+/H+ exchangers, Na+/K+ exchangers, NKKC or calcium channels.

A substance capable of modulating intracellular calcium levels may be any molecule having an inhibitory effect on solute carrier family 12 member 1, on solute carrier family 12 member 1,2,4 or on solute carrier family 12 member 1,2,4, 5.

Likewise, a substance capable of modulating intracellular calcium levels may be a molecule having an inhibitory effect on voltage-dependent calcium channels, preferably on voltage-dependent L-type, N-type or T-type calcium channels. In a preferred embodiment, the substance capable of modulating intracellular calcium levels may be a molecule having an inhibitory effect on voltage dependent L-type calcium channel subunits beta-1/ -4, subunit beta-2 or subunits alpha-1/delta-2.

According to the invention, the substance capable of modulating intracellular calcium levels is a substance that inhibits the NKCC co-transporter. NKCC co-transporters have been shown to promote rise in intracellular calcium either alone or in combination with other ions exchangers, such as Na/Ca exchanger, Na+/H+ exchanger, Na+/K+ exchanger (Liu et al. J Neurochem. 2010 September 1; 114(5): 1436-1446, ER Ca2+ signaling and mitochondrial Cyt c release in astrocytes following oxygen and glucose deprivation).

According to the invention, the substance capable of modulating intracellular calcium levels is a diuretic. Substances capable of modulating intracellular calcium levels include antiarrythmic agents. The skilled person is well aware of which drug or pharmaceuticals fall under the terms "diurectic" and "antiarrythmic agent".

Substances capable of modulating intracellular calcium levels include pyridinesulfonamides, in particular torasemide; isoindolines, in particular chlorthalidone; chlorophenoxyacetates, in particular etacrynic acid; aminobenzenesulfonamides, in particular furosemide and bumetanide; 4-substituted-3-amino-5-sulfamoylbenzoic acid derivatives, in particular but not limited to bumetanide, AqB007, AqB011 (Kourghi, M., Pei, J. V., De leso, M. L., Flynn, G., & Yool, A. J. (2016). Bumetanide Derivatives AqB007 and AqB011 Selectively Block the Aquaporin-1 Ion Channel Conductance and Slow Cancer Cell Migration. Molecular pharmacology, 89(1), 133-140), PF-2178, BUM13, BUM5 (Lykke, K., Töllner, K., Römermann, K., Feit, P. W., Erker, T., MacAulay, N., & Löscher, W. (2015). Structure-activity relationships of bumetanide derivatives: correlation between diuretic activity in dogs and inhibition of the human NKCC2A transporter. British journal of pharmacology, 172(18), 4469-4480), bumepamine; 1,2,4-benzothiadiazine-1,1-dioxides, in particular trichlormethiazide, hydroflumethiazide and methylclothiazide; dimethoxybenzenes, in particular ibutilde or verapamil; tetralins, in particular mibefradil; aryl-phenylketones, in particular dronedarone and amiodarone; 1,4-dihydropyridine and derivatives, in particular amlodipine, felodipine, diperdipine, nifedipine nimodipine, nisoldipine, nitrendipine, clevidipine, nicardipine and nilvadipine; benzoxadiazoles, preferably dihydropyridinecarboxylic acids and derivatives, in particular isradipine; diphenylmethylpiperazine and derivatives in particular flunarizine; heteroarylpiperidine in particular pimozide, domperidone; piperidines in particular cyproheptadine, fentanyl, alfentanil, sufentanil, flecainide, loperamide, methylphenidate, paroxetine, pempidine, perhexiline; benzocycloheptapyridine in particular loratadine; organic heterotetracyclic compound in particular nicergoline; aminoglycoside in particular, neomycin, gentamycin, kanamycin; piperidinecarboxamide in particular mepivacaine, bupivacaine; triptamines in particular indoramin; sulfonamides in particular clopamide; aminobenzamides in particular cisapride; aminopyrimidine in particular minoxidil; piperidine alkaloid in particular lobeline; piperidine antibiotic in particular cycloheximide non-proteinogenic amino acids such as gamma amino acids and derivatives, in particular gabapentin; piperidinemonocarboxylic acid in particular nipecotic acid, pipecolics acid; diarylmethane in particular penfluridol; benzothiazepine derivatives in particular diltiazem; phthalamides in particular thalidomide; diarylmethane in particular terfenadine, lomerazine, aromatic amine in particular ambroxol hydrochloride.

In another preferred embodiment, the substance capable of modulating intracellular calcium levels is selected from the group consisting of nifedipine, niludipine, nicardipine, nimodipine, NZ-105, amlodipine, felodipine, isradipine, diperdipine, emopamil, devapamil, verapamil, diltiazem, flunarizine, fluspirilene, pimozide, fantofarone, nicergoline, neomycin, gentamycin, kanamycin, cisapride, clopamide, cyproheptadine, loratadine, domperidone, fentanyl, alfentanil, sufentanil, flecainide, indoramin, isonepecotic acids, ketotifen, lobeline, loperamide, mepivacaine, methylphenidate, minoxidil, nipecotic acid, paroxetine, pempidine, penfluridol, perhexiline, pipecolics acid, bupivacaine, cyclohexemide, thalidomide, terfenadine, trihexyphenidyl, clevidipine, lomerazine, fostedil, anipamil, torasemide, chlorthalidone, etacrynic acid, furosemide, trichlormethiazide, hydroflumethiazide, methylclothiazide, bumetanide, ibutilde, mibefradil, dronedarone, amiodarone, nisoldipine, nitrendipine, nilvadipine, gabapentine, ambroxol hydrochloride.

In a non-claimed embodiment, the pharmaceutical composition or kit according to the present invention may additionally comprise a retinoic acid-related orphan receptor-alpha (RORA) agonist.

RORA is a ligand dependent orphan nuclear hormone receptor that, in combination with co-regulator proteins, serves as a transcriptional regulator. Recent studies have demonstrated a correlation between RORA and ASD, e.g., reduced expression of RORA in lymphoblastoid cell lines (LCL) derived from individuals with autism (Hu VW et al. Gene expression profiling differentiates autism case-controls and phenotypic variants of autism spectrum disorders: evidence for circadian rhythm dysfunction in severe autism. Autism Res. 2009 April; 2(2): 78-97); increased methylation leading to reduced expression of RORA in the LCL from ASD cases vs. sibling controls, and decreased expression of RORA protein in the prefrontal cortex and the cerebellum of individuals with autism (Nguyen A et al; Global methylation profiling of lymphoblastoid cell lines reveals epigenetic contribution to autism candidate gene RORA, whose protein product is reduced in autistic brain; FASEB J.2010 Aug;24(8):3036-41). Together these results link these molecular changes in RORA in blood-derived peripheral cells to molecular pathology in the brain tissues of individuals with autism (Tewarit Sarachana, Valerie W Hu. Genome wide identification of transcriptional targets of RORA reveals direct regulation of multiple genes associated with autism spectrum disorder. Molecular Autism May 2013, 4:14.). Consequently, in induction of RORA may be useful for treating symptoms in ASD patients, particular in ASD phenotype 1 patients.

It is disclosed herein that RORA agonists may be used for modulating intracellular calcium and thereof can maintain the effect of PDE 4 inhibition treatments.

RORA agonists may be phenylnaphthalenes, in particular adaptalene; sesquiterpenoids, in particular all-trans acitretin; diterpenoids, in particular alitretinoin or isotretinoin; thiochromanes, in particular tazarotene, retinoid esters, in particular etretinate; retinobenzoic acids, in particular tamibarotene, all-trans-retinoic acid or tretinoin.

The RORA agonist may be selected from the group consisting of adapalene, all-trans retinoic acid, tretinoin, vitamin A, all-trans acitretin, alitretinoin, tazarotene, etretinate, isotretinoin, tamibarotene, LGD-1550, AM580 and Ch 55.

By combining a substance capable of raising intracellular cAMP levels with a substance capable of modulating intracellular calcium levels, the long-lasting therapeutic effect of PDE4 inhibition can be maintained. Effect of monotherapy of ASD with bumetanide as described in WO 2011/086126 A1 takes between 3 weeks and 3 months. In contrast thereto, in the pharmaceutical composition or the kit according to the present invention, the effect of a substance capable of modulating intracellular calcium concentration such as bumetanide sets in within several days due to its indirect effect on modulating intracellular Ca2+ (Chen et al. (2008) Endoplasmic reticulum Ca2+ dysregulation and endoplasmic reticulum stress following in vitro neuronal ischemia: role of Na+-K+-Cl--cotransporter. J. Neurochem. 106:1563-1) compared to the CI- effect described in WO 2011/086126 A1.

It is also envisaged by the present invention that the pharmaceutical composition according to the present invention additionally comprises pharmaceutically acceptable carriers or excipients such as lubricants, disintegrants, antiadherents, binders, preservatives, sorbents or vehicles.

In a non-claimed aspect, the pharmaceutical composition or kit according to the present invention may additionally comprise an agent that inhibits cell proliferation. In another aspect, the present invention relates to a pharmaceutical composition for use in the treatment of ASD phenotype 1, the pharmaceutical composition comprising a substance capable of raising intracellular cAMP levels.

In one embodiment, the substance capable of raising intracellular cAMP levels is preferably a PDE inhibitor. In a particularly preferred embodiment, the PDE inhibitor is ibudilast. Ibudilast may be administered to the patient at a daily total dosage ranging from about 1-150 mg, preferably from about 5-80 mg, and more preferably from about 15-50 mg, divided among one, two, or three doses. It may be administered orally once, twice, or thrice daily to the patient using a dosage form that comprises 1, 5, 10, 15, 20, 25, 30, 35, 40, 45, or 50 mg ibudilast or a pharmaceutically acceptable salt thereof.

According to the invention, the substance capable of modulating Ca2+ is a NKKC1 inhibitor and the NKKC1 inhibitor is bumetanide. Bumetanide may be administered to the patient at a daily total dosage ranging from about 0.5 to 10 mg, preferably from 1 to 6 mg, and more preferably from 2 mg to 4 mg, divided into one, two, or three doses. It may be administered orally once, twice, or thrice daily to the patient using a dosage form that comprises 0.5, 1, 2 mg bumetanide, or a pharmaceutically acceptable salt thereof. Administration of a single dose may enhance patient compliance, while administration of several smaller doses ensures constant serum levels.

In a non-claimed aspect, the pharmaceutical composition or kit according to the present invention may additionally comprise an agent that inhibits cell proliferation, such as compounds targeting PI3K, AKT, mTOR, MAPK, ERK/JNK-P38 known to modulate cellular proliferation.

In a non-claimed aspect, the present invention relates to a pharmaceutical composition for use in the treatment of ASD in a patient, comprising a substance capable of raising intracellular cAMP levels, and a substance capable of modulating intracellular calcium levels, wherein the treatment comprises
∘ determining whether the patient suffers from ASD phenotype 1 and
∘ administering a therapeutically effective amount of the pharmaceutical composition to the patient if the patient suffers from ASD phenotype 1,
   wherein determining whether the patient suffers from ASD phenotype 1 includes at least one selected from
∘ subjecting the patient to a challenge test with Nrf2-activator,
∘ verifying for clinical signs of heightened expression of proliferation associated pathways,
∘ verifying for upregulation of Nrf2, or
∘ verifying for low blood levels of protein kinase A; and
wherein it is determined that the patient suffers from ASD phenotype 1 if he shows at least one of the following: negative behavioral response in a challenge test with a Nrf2 activator such as sulforaphane; clinical signs of heightened expression of proliferation-associated pathways; upregulation of *Nrf2* or low levels of protein kinase A.

In a non-claimed aspect, the pharmaceutical composition or kit according to the invention may be used for the treatment of Fragile X.

In a non-claimed aspect, a method is disclosed for treating a patient diagnosed with Fragile X Syndrome comprising administering to a patient in need thereof an effective amount of ibudilast or a pharmaceutically acceptable salt thereof.

In a non-claimed aspect, a method is disclosed for treating a patient diagnosed with autism spectrum disorder (ASD) comprising administering to a patient in need thereof an effective amount of ibudilast or a pharmaceutically acceptable salt thereof.

In a non-claimed aspect, a method is disclosed in which the patient exhibits characteristics consistent with being an ASD subtype 1 patient.

In a non-claimed aspect, a method is disclosed in which ibudilast or a pharmaceutically acceptable salt is administered to the patient at a daily total dosage ranging from about 1-150 mg, preferably from about 10-80 mg, and more preferably from about 15-50 mg, divided among one, two, or three doses.

In a non-claimed aspect, a method is disclosed in which ibudilast or a pharmaceutically acceptable salt thereof is administered orally once, twice, or thrice daily to the patient using a dosage form that comprises 1, 5, 10, 15, 20, 25, 30, 35, 40, 45, or 50 mg ibudilast or a pharmaceutically acceptable salt thereof.

### Example

A pharmaceutical composition comprising ibudilast and bumetanide was administered to patients with a diagnosis of autism spectrum disorder as defined in the DSM-5, or PDD-NOS or Asperger syndrome as defined in the DSM-IV.

Eligibility criteria: male sex, age 5-50, no current chronic illness, no history of active seizures within 1 year, normal liver, renal and thyroid function. Concomitant medications were permitted if doses were stable for at least 60 days prior to the initiation of the challenge test. Patients with no syndromic or identified genetic etiology.

Individuals with idiopathic ASD were classified as Phenotype 1 is they showed:
- at least 1 mandatory characteristics:
   ∘ enlarged head size versus control population characterized by at least one standard deviations above the mean head circumference at 24 months and/or formal macrocephaly (HC>97% of the general population)
      and/or
   ∘ cyclical aggravation of core or ancillary autism symptoms potentiated by periods of infectious events, deciduous tooth loss, post-traumatic injury, endogenous and exogenous temperature variation
      and
- at least 2, and most preferably at least 3 of the following 20 characteristics:
   ∘ Accelerated hair and nail growth versus control population
   ∘ Increased tendency to present with lighter colors of skin and eyes as compared to individuals of the same ethnicity
   ∘ Substantially longer eyelashes than control subjects of the same ethnicity
   ∘ At least 5 non-contiguous areas of hypopigmented skin, particularly presenting on the back of the patient
   ∘ Signs of edema during periods of infectious events, deciduous tooth loss, post-traumatic injury, or endogenous and exogenous factors modifying body temperature; more specifically, facial edema located in the periorbital and forehead areas
   ∘ Increased blood levels of gamma-glutamyl transpeptidase (GGT) as compared to typically developing individuals of the same age and ethnicity
   ∘ Congenital genitourinary malformations and/or functional impairment to initiate urinating
   ∘ Hypoplasia of the patella
   ∘ Frequent episodes of diarrhea specifically before the age of 5 years ∘ Frequent episodes of tinnitus
   ∘ Thinning of the corpus callosum
   ∘ Positive family history for hematological malignancies in particular but not limited to myeloma and acute promyelocytic leukemia
   ∘ Positive family history for rheumatoid arthritis, that is at least two affected first-degree relatives in two consecutive generations
   ∘ Adverse events in response to acetyl-salicylic acid or its derivatives
   ∘ Iris coloboma, either monolateral or bilateral
   ∘ Sleep hyperhidrosis particularly in newborns, toddlers and young children (notably increased night sweating during infancy and childhood -often reported by relatives to requires bed linen changes)
   ∘ Increased Th1/Th2 ratio (i.e. elevated levels of Interleukin 1 beta, Interleukin 6, TNF-alpha, Interferon gamma)
   ∘ Congenital accessory or duplicated spleen
   ∘ Congenital absence of the cisterna chyli
   ∘ Reported history of mother suffering from viral or bacterial infection during pregnancy and/or biologically confirmed Maternal immune activation during pregnancy

### First Intervention:

Description: Four of the patients characterized as above were administered ibudilast. The four subjects had varying levels of functioning ranging from mild to severe, and varying different levels of verbal and intellectual abilities. IQ values ranged from 61 to 86. All were males.

**Table 1: Patient status prior to intervention**

| ADI baseline score | Patient 1 | Patient 2 | Patient 3 | Patient 4 |
|---|---|---|---|---|
| ADI-r SI | 28 | 20 | 14 | 27 |
| ADI-r C | 24 | 19 | 18 | 17 |
| ADI-r RI | 7 | 5 | 7 | 7 |

Four patients were treated with ibudilast at a daily total dosage of 0,6 mg/kg, TID.

**Table 2: Patient status after treatment for four weeks of treatment with ibudilast.**

| ADI baseline score | Patient 1 | Patient 2 | Patient 3 | Patient 4 |
|---|---|---|---|---|
| ADI-r SI | 21 | 16 | 12 | 22 |
| ADI-r C | 20 | 15 | 14 | 14 |
| ADI-r RI | 6 | 5 | 5 | 7 |

After between two and four months of mono-treatment with ibudilast, loss of treatment efficacy was reported in all four patients with gradual return to pre-treatment baseline scores.

A new treatment regimen was introduced, this time ibudilast at a daily total dosage of 0.6 mg/kg, TID, was administered in conjunction with bumetanide at a daily total dosage of 0.08 mg/kg, BID (with a maximum upper daily dose of 2 mg/kg/day).

Following a mean length of administration of 8 days of bi-therapy treatment effect on reduction of ADI-r scores reflected restoration of initial monotherapy efficacy.

**Table 3: Patient status after treatment for 8 days with ibudilast + bumetanide**

| ADI baseline score | Patient 1 | Patient 2 | Patient 3 | Patient 4 |
|---|---|---|---|---|
| ADI-r SI | 20 | 15 | 11 | 22 |
| ADI-r C | 20 | 13 | 14 | 14 |
| ADI-r RI | 7 | 5 | 6 | 5 |

## Claims

1. A pharmaceutical composition comprising
∘ a substance capable of raising intracellular cAMP levels and
∘ a substance capable of modulating intracellular calcium concentration,
wherein the substance capable of raising intracellular cAMP levels is ibudilast and the substance capable of modulating intracellular calcium concentration is bumetanide.

2. The composition according to claim 1 for use in the treatment of autism spectrum disorder (ASD).

3. A kit for use in the treatment of ASD comprising
∘ a substance capable of raising intracellular cAMP levels and
∘ a substance capable of modulating intracellular calcium concentration,
wherein the substance capable of raising intracellular cAMP levels is ibudilast and the substance capable of modulating intracellular calcium concentration is bumetanide.

## Patentansprüche

1. Pharmazeutische Zusammensetzung, umfassend
• eine Substanz, die den intrazellulären cAMP-Spiegel erhöhen kann und
• eine Substanz, die die intrazelluläre Calciumkonzentration modulieren kann,
wobei die Substanz, die die intrazellulären cAMP-Spiegel erhöhen kann, Ibudilast ist und die Substanz, die die intrazelluläre Calciumkonzentration modulieren kann, Bumetanid ist.

2. Zusammensetzung nach Anspruch 1 zur Verwendung bei der Behandlung von Autismus-Spektrum-Störung (ASS).

3. Ein Kit zur Verwendung bei der Behandlung von ASS, umfassend
• eine Substanz, die den intrazellulären cAMP-Spiegel erhöhen kann und
• eine Substanz, die die intrazelluläre Calciumkonzentration modulieren kann,
wobei die Substanz, die die intrazellulären cAMP-Spiegel erhöhen kann, Ibudilast ist und die Substanz, die die intrazelluläre Calciumkonzentration modulieren kann, Bumetanid ist.

## Revendications

1. Composition pharmaceutique comprenant
• une substance capable d'augmenter les niveaux d'AMPc intracellulaire et
• une substance capable de moduler la concentration de calcium intracellulaire,
dans laquelle la substance capable d'élever les niveaux d'AMPc intracellulaire est l'ibudilast et la substance capable de moduler la concentration de calcium intracellulaire est le bumétanide.

2. Composition selon la revendication 1 pour son utilisation dans le traitement des troubles du spectre autistique (TSA).

3. Kit destiné à être utilisé dans le traitement des TSA comprenant
• une substance capable d'augmenter les niveaux d'AMPc intracellulaire et
• une substance capable de moduler la concentration de calcium intracellulaire,
dans lequel la substance capable d'élever les niveaux d'AMPc intracellulaire est l'ibudilast et la substance capable de moduler la concentration de calcium intracellulaire est le bumétanide.
